# EUROPEAN PATENT APPLICATION

(11) **EP 4 410 293 A1**
(43) Date of publication of application: **07.08.2024**
(21) Application number: 22878808.9
(22) Date of filing: 28.09.2022
(51) Int. Cl.: A61K 31/536, A61K 31/138, A61K 45/06, A61P 31/12, A61P 25/24

(54) **PHARMACEUTICAL COMPOSITION FOR PREVENTING OR TREATING VIRAL INFECTIOUS DISEASES, CONTAINING EFAVIRENZ AND FLUOXETINE AS ACTIVE INGREDIENTS**

(30) Priority: 06.10.2021 KR 20210132701
(71) Applicant: Medience Co., Ltd., Chuncheon-si, Gangwon-do 24232 (KR)
(72) Inventor: PARK, Soo Hyun, Chuncheon-si Gangwon-do 24267 (KR); KIM, Set Byeol, Chuncheon-si Gangwon-do 24447 (KR); KIM, Jeong Hoon, Chuncheon-si Gangwon-do 24277 (KR); MO, Young Won, Chuncheon-si Gangwon-do 24221 (KR)
(74) Representative: RGTH
(86) International application number: PCT/KR2022/014517
(87) International publication number: WO 2023/058987

(57) **Abstract**

The present invention relates to a pharmaceutical composition for preventing or treating viral infectious diseases, containing efavirenz and fluoxetine as active ingredients. It has been identified that when a complex, mixture or combination of efavirenz and fluoxetine is administered or a complex, mixture or combination of efavirenz and fluoxetine, to which a biguanide-based compound metformin is added, is administered, antiviral efficacy against various zoonotic viruses including SARS-CoV-2 is remarkably higher than when each is administered alone, and thus efavirenz and fluoxetine, in addition to the biguanide-based compound, can be effectively used as active ingredients of a composition for preventing or treating viral infectious diseases.

## Description

### [Technical Field]

The present invention relates to a pharmaceutical composition for preventing or treating viral infectious diseases, containing a first ingredient containing efavirenz and a second ingredient containing fluoxetine as active ingredients of a complex, mixed, or combined preparation. In addition, the present invention relates to a pharmaceutical composition for preventing or treating viral infectious diseases containing a first ingredient containing efavirenz, a second ingredient containing fluoxetine, and a third ingredient containing a biguanide-based compound or a pharmaceutically acceptable salt thereof as active ingredients of a complex, mixed, or combined preparation.

### [Background Art]

Coronavirus disease 2019 (COVID-19) is an acute respiratory disease caused by the type 2 severe acute respiratory syndrome coronavirus (SARS-CoV-2), which is a newly discovered beta-coronavirus, and shows a variety of clinical manifestations and mortality rates, ranging from asymptomatic to acute symptoms such as fever, fatigue, sore throat, dry cough, difficulty breathing, and diarrhea, pneumonia with acute respiratory distress syndrome, and multi-organ failure. After COVID-19 was first found in 2019, as of September 2021, more than 210 million people around the world have been infected and more than 4.55 million people died. It is known that COVID-19 has a high infectivity of 60% or more even before symptoms appear or in a completely asymptomatic state, unlike the usual case where infection occurs after symptoms appear, quickly spread even when the infected person is not aware of it, and is thus even more difficult to control. Currently, group immunity through vaccination and the development of effective therapeutic agents for COVID-19 are considered the only solutions to block the spread and minimize severe cases and deaths.

A zoonosis, including COVID-19, refers to an infectious disease caused by pathogens transmitted between animals and humans. There are known worldwide several hundreds of types of pathogens of zoonosis including viruses, rickettsia, chlamydia, bacteria, protozoa, and parasites, and various hosts and vectors having the pathogens. It is known that 75% of major infectious disease epidemics that have occurred since 2000 are transmitted from animals to humans, and zoonosis is caused by increased opportunities for contact between humans and animals due to the expansion of artificial habitats, increased pathogenicity due to genetic mutation and recombination of pathogens and interspecies transmission caused by environmental destruction and climate change, and changes in host specificity. Zoonosis is characterized in that pathogens are transmitted directly to livestock or humans due to unexpected infections in wild animals and wild birds, or spread more rapidly due to the development of transportation and increased travel and trade. Like COVID-19 pandemic, zoonosis has a risk of causing astronomical social and economic losses. Therefore, there is an urgent need for research to introduce novel methods of preventing or treating a variety of zoonoses.

Antiviral agents are drugs that treat infectious diseases caused by viruses by weakening or eliminating the action of viruses that invade the human body, and have the effect of suppressing the proliferation of viruses. Depending on the mechanism of action thereof, antiviral agents may be classified into inhibitors for virus attachment and penetration and inhibitors for virus proliferation. Most antiviral agents exhibit antiviral action by inhibiting various types of enzymes required for virus proliferation.

For example, efavirenz, which has been approved by the FDA, binds directly to the hydrophobic pocket near the active site of the human immunodeficiency virus (HIV) reverse transcriptase to change the structure of the enzyme and thereby inhibit the enzymatic action thereof.

In addition, antidepressants are drugs that ameliorate depressive symptoms by controlling the imbalance of neurotransmitters (serotonin, norepinephrine, dopamine) related to depression, and may be classified into tricyclic antidepressants (TCAs), monoamine oxidase inhibitors (MAOIs), selective serotonin reuptake inhibitors (SSRIs), serotonin-norepinephrine reuptake inhibitors (SNRls) and the like.

Tricyclic antidepressants are substances that ameliorate depressive symptoms by blocking the reuptake of serotonin and norepinephrine at the terminals of nerve cells to increase the concentration of neurotransmitters within the synapse, and selective serotonin reuptake inhibitors (SSRIs) are substances that ameliorate depressive symptoms by selectively blocking serotonin reuptake at nerve cell terminals to increase the concentration of serotonin within the synapse and increase the activity of serotonin. Selective serotonin reuptake inhibitors are most commonly used as antidepressants because they are superior to tricyclic antidepressants in terms of side effects and safety.

Recently, the therapeutic effects on COVID-19 of these antidepressants have been reviewed. For example, clomipramine, a tricyclic antidepressant, has been reported to be effective in preventing SARS-CoV-2 from invading cells and suppressing viral proliferation even after invasion, and fluvoxamine, a selective serotonin reuptake inhibitor, has been reported to be effective in blocking cytokine storm, a major cause of death in COVID-19 patients.

Accordingly, the present inventors have conducted research to develop a combination of substances having superior antiviral effects against various zoonotic viruses, including SARS-CoV-2, and as a result, the present inventors found that a combination of efavirenz, as an antiviral agent, and fluoxetine, as an antidepressant, according to the present invention exhibits a significant synergistic effect in inhibiting the proliferation of zoonotic viruses, and developed a novel mixed, complexed or combined antiviral agent.

### [Prior art literature]

### [Non-patent literature]

Y Kato et al., Clomipramine suppresses ACE2-mediated SARS-CoV-2 entry, https://doi.org/10.1101/2021.03.13.435221
EJ Lenze et al., Fluvoxamine vs Placebo and Clinical Deterioration in Outpatients With Symptomatic COVID-19: A Randomized Clinical Trial, JAMA. 2020;324(22):2292-2300

### [Disclosure]

### [Technical Problem]

Developing, approving, and launching a completely new drug requires a lot of time and money to conduct preclinical trial and three-stage clinical trials to prove the effects, side effects, and harmfulness of drugs. Drug repurposing using existing drugs that have already proven to be safe and effective has emerged as a solution to dramatically reduce the exponential cost of new drug development. It is expected that drug repurposing can greatly reduce clinical trial costs and has a low risk of side effects, and are quickly applicable to patients even in emergency situations such as the COVID-19 pandemic.

Therefore, it is an object of the present invention to provide a composition for preventing or treating viral infectious diseases that contain, conventional pharmaceuticals, efavirenz and fluoxetine as active ingredients and exhibit excellent antiviral activity.

### [Technical Solution]

The present invention is based on the finding that a pharmaceutical composition for preventing or treating viral infectious diseases containing a first ingredient containing efavirenz and a second ingredient containing fluoxetine as active ingredients, as a complexed, mixed or combined preparation for preventing or treating viral infectious diseases, has an effect of remarkably inhibiting proliferation of viruses. In accordance with an aspect of the present invention, the above and other objects can be accomplished by the provision of a pharmaceutical composition containing the first component and the second component as active ingredients of the complexed, mixed, or combined preparation.

In accordance with another aspect of the present invention, provided is a pharmaceutical composition for preventing or treating viral infectious diseases containing a first ingredient containing efavirenz, a second ingredient containing fluoxetine, and a third ingredient containing a biguanide-based compound or a pharmaceutically acceptable salt thereof as active ingredients, as a complexed, mixed or combined preparation for preventing or treating viral infectious diseases.

In accordance with another aspect of the present invention, provided is a complexed, mixed or combined preparation kit for preventing or treating viral infectious diseases containing a preparation containing efavirenz and a preparation containing fluoxetine.

In accordance with another aspect of the present invention, provided is a complexed, mixed or combined preparation kit for preventing or treating viral infectious diseases containing a preparation containing efavirenz, a preparation containing fluoxetine, and a preparation containing a biguanide-based compound or a pharmaceutically acceptable salt thereof.

In accordance with another aspect of the present invention, provided is a method of preventing or treating viral infectious diseases including administering a complex, mixture or combination of a first ingredient containing efavirenz and a second ingredient containing fluoxetine to a subject.

In accordance with another aspect of the present invention, provided is a method of preventing or treating viral infectious diseases including administering a complex, mixture or combination of a first ingredient containing efavirenz, a second ingredient containing fluoxetine, and a third ingredient containing a biguanide-based compound or a pharmaceutically acceptable salt thereof to a subject.

In accordance with another aspect of the present invention, provided is the use of a complexed, mixed or combined preparation containing a first ingredient containing efavirenz and a second ingredient containing fluoxetine as active ingredients, as a pharmaceutical composition for preventing or treating viral infectious diseases.

In accordance with another aspect of the present invention, provided is the use of a complexed, mixed or combined preparation containing a first ingredient containing efavirenz, a second ingredient containing fluoxetine, and a third ingredient containing a biguanide-based compound or a pharmaceutically acceptable salt thereof as active ingredients, as a pharmaceutical composition for preventing or treating viral infectious diseases.

### [Advantageous effects]

Treatment with the complexed, mixed, or combined preparation of efavirenz and fluoxetine according to the present invention exhibits significantly synergistically effective antiviral efficacy against zoonotic viruses, including SARS-CoV-2, compared to treatment with either efavirenz or fluoxetine alone, and thus a pharmaceutical composition containing the complexed, mixed, or combined preparation of efavirenz and fluoxetine as an active ingredient may be useful for preventing or treating viral infectious diseases.

Further, treatment with the complexed, mixed, or combined preparation of metformin, as a biguanide compound, as well as efavirenz and fluoxetine according to the present invention exhibits significantly synergistically effective antiviral efficacy against zoonotic viruses, including SARS-CoV-2, compared to treatment with metformin, efavirenz or fluoxetine alone, treatment with a complexed, mixed, or combined preparation of metformin and efavirenz, or treatment with a complexed, mixed, or combined preparation of metformin and fluoxetine, and thus a pharmaceutical composition containing the complexed, mixed, or combined preparation of metformin, efavirenz and fluoxetine as an active ingredient may be useful for preventing or treating viral infectious diseases.

### [Description of Drawings]

FIG. 1 shows the relative copy number of the N genes of SARS-CoV-2 resulting from qPCR of Vero E6 (Vero) cells treated with 1.5 µM efavirenz (Efa), 1 µM fluoxetine (Flu), or 2.5 mM metformin (Met) alone, or a combination of 1.5 µM efavirenz (Efa), 1 µM fluoxetine (Flu), and 2.5 mM metformin (Met) after SARS-CoV-2 infection.
FIG. 2 shows the relative copy number of the N genes of HCoV-OC43 resulting from qPCR of Vero E6 (Vero) cells treated with a combination of 1.5 µM efavirenz (Efa) and 1 µM fluoxetine (Flu) after HCoV-OC43 infection.
FIG. 3 shows the relative copy number of the N genes of SEOV resulting from qPCR of Vero E6 (Vero) cells treated with a combination of 1.5 µM efavirenz (Efa) and 1 µM fluoxetine (Flu) after SEOV infection.
FIG. 4 shows the relative copy number of the N genes of VACV resulting from qPCR of Vero E6 (Vero) cells treated with a combination of 1.5 µM efavirenz (Efa) and 1 µM fluoxetine (Flu) after VACV infection.
FIG. 5 shows the relative copy number of the N genes of ZIKV resulting from qPCR of Vero E6 (Vero) cells treated with a combination of 1.5 µM efavirenz (Efa) and 1 µM fluoxetine (Flu) after ZIKV infection.

### [Best Mode]

Hereinafter, the present invention will be described in more detail.

The present invention provides a pharmaceutical composition for preventing or treating viral infectious diseases containing a first ingredient containing efavirenz, a second ingredient containing fluoxetine as active ingredients, as a complexed, mixed or combined preparation for preventing or treating viral infectious diseases, a method of preventing or treating viral infectious diseases including administering a complex, mixture or combination of a first ingredient containing efavirenz and a second ingredient containing fluoxetine to a subject, and the use of a complexed, mixed or combined preparation containing a first ingredient containing efavirenz and a second ingredient containing fluoxetine as active ingredients, as a pharmaceutical composition for preventing or treating viral infectious diseases.

The present invention provides a pharmaceutical composition for preventing or treating viral infectious diseases containing a first ingredient containing efavirenz, a second ingredient containing fluoxetine, and a third ingredient containing a biguanide-based compound or a pharmaceutically acceptable salt thereof as active ingredients, as a complexed, mixed or combined preparation for use in preventing or treating viral infectious diseases, a method of preventing or treating viral infectious diseases including administering a complex, mixture or combination of a first ingredient containing efavirenz, a second ingredient containing fluoxetine, and a third ingredient containing a biguanide-based compound or a pharmaceutically acceptable salt thereof to a subject, and the use of a complexed, mixed or combined preparation containing a first ingredient containing efavirenz, a second ingredient containing fluoxetine, and a third ingredient containing a biguanide-based compound or a pharmaceutically acceptable salt thereof as active ingredients, as a pharmaceutical composition for preventing or treating viral infectious diseases.

As used herein, the term "prevention" refers to any action that suppresses or delays the onset of diseases by administration of the pharmaceutical composition according to the present invention. As used herein, the term "amelioration" or "treatment" refers to any action that ameliorates or beneficially changes symptoms due to diseases by administration of the pharmaceutical composition according to the present invention.

As used herein, the term "administration" means supplying the pharmaceutical composition of the present invention to a patient using any suitable method. The pharmaceutical composition of the present invention may be administered orally or parenterally through any general route enabling the pharmaceutical composition to reach the target site. In addition, the composition may be administered by any device enabling the active ingredients to delivering to target cells.

The biguanide-based compound according to the present invention may be selected from the group consisting of metformin, phenformin, buformin, and biguanide.

In one embodiment of the present invention, the concentration of efavirenz may be 0.001 µM to 10 mM and the concentration of fluoxetine may be 0.001 µM to 10 mM.

In one embodiment of the present invention, the concentration of the biguanide-based compound or pharmaceutically acceptable salt thereof that may be further added may be 0.01 µM to 100 mM.

In one embodiment of the present invention, the content of the efavirenz, fluoxetine, and biguanide-based compound or pharmaceutically acceptable salt thereof in the pharmaceutical composition of the present invention may be appropriately selected depending on the type of preparation or the like.

In one embodiment of the present invention, when efavirenz, fluoxetine and a biguanide-based compound or a pharmaceutically acceptable salt thereof are formulated into a single preparation, the content of efavirenz is generally about 0.01 to about 99.99 wt%, specifically about 0.01 to about 90 wt%, preferably about 0.1 to about 90 wt%, more preferably about 0.1 to about 80 wt%, even more preferably about 0.1 to about 70 wt%, with respect to the total weight of the preparation, the content of fluoxetine is generally about 0.01 to about 99.99 wt%, specifically about 0.01 to about 90 wt%, preferably about 0.1 to about 80 wt%, and more preferably about 0.1 to about 70 wt%, even more preferably, about 0.1 to about 60 wt%, with respect to the total weight of the preparation, and the content of the biguanide-based compound or the pharmaceutically acceptable salt thereof is generally about 0.00001 to about 99.99 wt%, specifically about 0.00001 to about 90 wt%, preferably about 0.0001 to about 90 wt%, more preferably about 0.0001 to about 80 wt%, and even more preferably about 0.0001 to about 70 wt%, with respect to the total weight of the preparation.

Meanwhile, when the first ingredient of efavirenz and the second ingredient of fluoxetine in the pharmaceutical composition of the present invention are blended into a single preparation, the first ingredient and the second ingredient may be blended in a weight (parts by weight) ratio of 0.0000001 to 10:1. In addition, when the first ingredient of efavirenz, the second ingredient of fluoxetine, and the third ingredient of the biguanide-based compound or the pharmaceutically acceptable salt thereof are blended into a single preparation, the first ingredient, the second ingredient, and the third ingredient may be blended in a weight (parts by weight) ratio of 0.0000001 to 10:1:0.01 to 100000.

In one embodiment of the present invention, when efavirenz, fluoxetine and a biguanide-based compound or a pharmaceutically acceptable salt thereof are separately prepared and combined, the content of efavirenz is generally about 0.01 to about 99.99 wt%, specifically about 0.1 to about 99.99 wt%, preferably about 0.1 to about 90 wt%, more preferably about 0.1 to about 80 wt%, even more preferably about 1 to about 80 wt%, with respect to the total weight of the preparation containing the same, the content of fluoxetine is generally about 0.01 to about 99.99 wt%, specifically about 0.1 to about 99.99 wt%, preferably about 0.1 to about 90 wt%, more preferably, about 0.1 to about 80 wt%, even more preferably, about 1 to about 80 wt%, with respect to the total weight of the preparation containing the same, and the content of the biguanide-based compound or the pharmaceutically acceptable salt thereof is generally about 0.01 to about 99.99 wt%, specifically about 0.1 to about 99.99 wt%, preferably about 0.1 to about 90 wt%, more preferably about 0.1 to about 80 wt%, and even more preferably about 1 to about 80 wt%, with respect to the total weight of the preparation containing the same.

Meanwhile, the efavirenz, the fluoxetine, and the biguanide-based compound or pharmaceutically acceptable salt thereof are separately prepared and used in combination, the content of additives such as carriers is variable, but is generally about 1 to 99.00 wt%, specifically about 1 to about 90 wt%, preferably about 10 to about 90 wt%, more preferably about 10 to about 80 wt%, and even more preferably about 10 to about 70 wt%, with respect to the total weight of each preparation containing the same.

The virus according to the present invention may be a zoonotic virus and may specifically be selected from the group consisting of *Betacoronavirus* and a subgenus thereof, *Orthohantavirus, Orthopoxvirus, Flavivirus, Lyssavirus* and *Bandavirus.*

Examples of the *Betacoronavirus* and subgenus thereof include, but are not limited to, *Bovine coronavirus, human coronavirus* OC43 (HCoV-OC43), *China Rattus coronavirus* (HKU24), human coronavirus HKU1 (HCoV-HKU1), *murine coronavirus* (M-CoV), *Myodes coronavirus* (2JL14), *severe acute respiratory syndrome coronavirus* 2 (SARS-CoV-2) and variants thereof, *middle east respiratory syndrome coronavirus* (MERS-CoV), *severe acute respiratory syndrome coronavirus 1* (SARS-CoV-1), severe acute respiratory syndrome-related coronavirus (SARSr-CoV), *Bat SARS-like coronavirus* WIV1 (bat SL-CoV-WIV1), *bat coronavirus* (RaTG13), *hedgehog coronavirus 1, Pipistrellus bat coronavirus* (HKU5), *Tylonycteris bat coronavirus* (HKU4), *Eidolon bat coronavirus* (C704), *Rousettus bat coronavirus* (GCCDC1), *Rousettus bat coronavirus* HKU9 (HKU9-1) and *Bat Hp-betacoronavirus* Zhejiang2013.

In addition, examples of the *Orthohantavirus* include, but are not limited to, *Andes orthohantavirus* (ANDV), *Asama orthohantavirus* (ASAV), *Asikkala orthohantavirus* (ASIV), *Bayou orthohantavirus* (BAYV), *Black Creek Canal orthohantavirus (*BCCV), *Bowe orthohantavirus* (BOWV), *Bruges orthohantavirus, Cano Delgadito orthohantavirus* (CADV), *Cao Bang orthohantavirus, Choclo orthohantavirus* (CHOV), *Dabieshan orthohantavirus, Dobrava-Belgrade orthohantavirus* (DOBV), *EI Moro Canyon orthohantavirus, Fugong orthohantavirus, Fusong orthohantavirus, Hantaan orthohantavirus* (HTNV), *Jeju orthohantavirus, Kenkeme orthohantavirus, Isla Vista virus* (ISLAV), *Khabarovsk orthohantavirus* (KHAV), *Laguna Negra orthohantavirus* (LANV), *Muleshoe virus* (MULV), *Luxi orthohantavirus, Maporal orthohantavirus, Montano orthohantavirus* (MTNV), *Necocli orthohantavirus, Oxbow orthohantavirus* (OXBV), *New York virus (*NYV), *Prospect Hill orthohantavirus* (PHV), *Puumala orthohantavirus* (PUUV), *Robina orthohantavirus, Rockport orthohantavirus* (RKPV), *Rio Mamore virus* (RIOMV), *Rio Segundo virus* (RIOSV), *Sangassou orthohantavirus* (SANGV), *Seewis orhtohantavirus, Seoul orthohantavirus* (SEOV), *Sin Nombre orthohantavirus* (SNV), *Tatenale orthohantavirus, Tigray orthohantavirus, Thailand orthohantavirus* (THAIV), *Thottapalayam virus* (TPMV), *Topografov virus* (TOPV), *Tula orthohantavirus* (TULV) and *Yakeshi orthohantavirus.*

In addition, examples of *Orthopoxvirus* include, but are not limited to, *Abatino macacapox virus, Akhmeta virus, Alaskapox virus,* Camelpox virus (CMLV), *Cowpox virus, Ectromelia virus, Monkeypox virus, Raccoonpox virus, Skunkpox virus, Taterapox virus, Vaccinia virus, Variola virus,* and *Volepox virus.*

In addition, examples of *Flavivirus* include, but are not limited to, *Apoi virus, Aroa virus, Bagaza virus, Banzi virus, Bouboui virus, Bukalasa bat virus, Cacipacore virus, Carey Island virus, Cowbone Ridge virus, Dakar bat virus, Dengue virus* (DENV), *Edge Hill virus, Entebbe bat virus, Gadgets Gully virus* (GGYV), *Ilheus virus, Israel turkey meningoencephalomyelitis virus, Japanese encephalitis virus* (JE), *Jugra virus, Jutiapa virus, Kadam virus, Kedougou virus* (KEDV), *Kokobera virus, Koutango virus, Kyasanur Forest disease virus* (KFD), *Langat virus* (LGTV), *Louping ill virus, Meaban virus, Modoc virus* (MODV), *Montana myotis leukoencephalitis virus, Murray Valley encephalitis virus* (MVEV), *Ntaya virus, Omsk hemorrhagic fever virus, Phnom Penh bat virus, Powassan virus* (POWV), *Rio Bravo virus, Royal Farm virus, Saboya virus, Saint Louis encephalitis virus, Sal Vieja virus, Son Perlita virus, Saumarez Reef virus, Sepik virus* (SEPV), *Tembusu virus, Tick-borne encephalitis virus* (TBEV), *Tyuleniy virus, Uganda 5 virus, Usutu virus* (USUV), *Wesselsbron virus, West Nile virus* (WNV), *Yaounde virus, Yellow fever virus, Yokose virus* (YOKV) and *Zika virus.*

In addition, examples of *Lyssavirus* include, but are not limited to, *Rabies lyssa virus.*

In addition, examples of *Bandavirus* include, but are not limited to, severe fever with thrombocytopenia syndrome virus (SFTSV).

In one embodiment of the present invention, the preparation is provided in a formulation selected from the group consisting of a tablet, a capsule, an injection, a troche, a powder, a granule, a solution, a suspension, an oral liquid, an emulsion, a syrup, a suppository, a vaginal tablet, and a pill, but is not limited thereto. The preparation may be provided in an appropriate formulation as needed.

In another aspect, the present invention is directed to a combined preparation kit for preventing or treating viral infectious diseases containing a complexed, mixed or combined preparation of efavirenz and fluoxetine.

In another aspect, the present invention is directed to a complexed, mixed or combined preparation kit for preventing or treating viral infectious diseases containing a preparation containing efavirenz, a preparation containing fluoxetine, and a preparation containing a biguanide-based compound or a pharmaceutically acceptable salt thereof.

In one aspect of the present invention, the efavirenz, the fluoxetine, the biguanide-based compound, the contents thereof, the content ratio, and viral infectious diseases are the same as in the description associated with the pharmaceutical composition for preventing or treating viral infectious diseases, and detailed descriptions thereof are omitted.

The efavirenz, the fluoxetine and the biguanide-based compound according to the present invention may be used in the form of a pharmaceutically acceptable salt. The salt is preferably an acid addition salt produced from a pharmaceutically acceptable free acid. The acid addition salt includes inorganic acids such as hydrochloric acid, nitric acid, phosphoric acid, sulfuric acid, hydrobromic acid, hydroiodic acid, nitrous acid or phosphorous acid, and non-toxic organic acids such as aliphatic mono- and di-carboxylates, phenyl-substituted alkanoates, hydroxy alkanoates and alkanedioates, aromatic acids, and aliphatic and aromatic sulfonic acids. Examples of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, hydroxybutyrate, glycolate, malate, tartrate, methanesulfonate, propanesulfonate, naphthalene-1-sulfonate, naphthalene-2-sulfonate and mandelate.

The acid addition salt according to the present invention may be prepared using a conventional method, for example, by dissolving the compound of the present invention in an excess of aqueous acid and precipitating the salt in a water-miscible organic solvent, such as methanol, ethanol, acetone or acetonitrile. In addition, the acid addition salt may be prepared by evaporating the solvent or excess acid from the resulting mixture, followed by drying, or by suction-filtering the precipitated salt.

In addition, the pharmaceutically acceptable metal salt may be prepared using a base. Alkali metal or alkaline earth metal salts are obtained, for example, by dissolving the compound in an excess of alkali metal hydroxide or alkaline earth metal hydroxide solution, filtering off the undissolved compound salt, and evaporating the filtrate, followed by drying. At this time, the metal salt is a sodium, potassium, or calcium salt which is pharmaceutically appropriate. Further, the silver salt is obtained by reacting an alkali metal or alkaline earth metal salt with an appropriate silver salt (e.g., silver nitrate).

When the composition is formulated, it is prepared using a diluent or excipient such as a commonly used filler, extender, binder, wetting agent, disintegrant, or surfactant.

Solid preparations for oral administration may include tablets, pills, powders, granules, capsules, and the like, and the solid preparations may be prepared by mixing the compound of the present invention with at least one excipient, for example, starch, calcium carbonate, sucrose, lactose or gelatin. In addition to simple excipients, lubricants such as magnesium stearate and talc may also be used. Liquid preparations for oral administration include suspensions, oral liquids, emulsions, syrups and the like, and various excipients such as wetting agents, sweetening agents, fragrances, preservatives, and the like, in addition to water and liquid paraffin, which are commonly used simple diluents, may be included.

Preparations for parenteral administration may include sterile aqueous solutions, non-aqueous solutions, suspensions, emulsions, lyophilizates, suppositories, and the like.

Non-aqueous solvents or suspension solvents may include propylene glycol, polyethylene glycol, vegetable oils such as olive oil, and injectable esters such as ethyl oleate. The base of the suppository may be Witepsol, macrogol, Tween 61, cacao butter, laurin, glycerogelatin, or the like.

The pharmaceutical composition of the present invention may be administered in a pharmaceutically effective amount. As used herein, the term "pharmaceutically effective amount" means an amount sufficient to treat or prevent a disease at a reasonable benefit/risk ratio applicable to pharmaceutical (medical) treatment, while causing no side effects. The effective dosage is determined depending on factors including the state of health of the patient, severity, drug activity, drug sensitivity, administration method, administration time, administration route, excretion rate, treatment period, drugs used in combination with or concurrently with the composition of the present invention, and other factors well known in the pharmaceutical field. The composition of the present invention may be administered as a single therapeutic agent or in combination with other therapeutic agents, sequentially or simultaneously with conventional therapeutic agents, and in one or multiple doses. Taking into consideration these factors, it is important to administer the composition in the minimum amount sufficient to achieve maximum efficacy without side effects, which can be easily determined by those skilled in the art.

Specifically, the effective amount of the compound according to the present invention may vary depending on the age, gender, and body weight of patients and is generally administered at 0.01 mg to 100 mg, preferably 0.1 mg to 10 mg, per kg of body weight every day or every other day. Alternatively, the effective amount may be administered in divided doses 1 to 3 times a day. However, the effective amount may increase or decrease depending on the route of administration, severity of disease, gender, weight, age, and the like and thus does not limit the scope of the present invention in any way.

In another aspect, a mixture, complex or combination of the first ingredient containing efavirenz, the second ingredient including fluoxetine, and the third ingredient including a biguanide-based compound or a pharmaceutically acceptable salt thereof is administered to subjects in need of treatment for viral infectious diseases. Various viral infectious diseases including infectious diseases of the viruses described above may be treated using the method according to the present invention.

In a specific embodiment of the present invention, in order to confirm the antiviral efficacy of a complexed, mixed or combined preparation of efavirenz, fluoxetine and the biguanide-based compound, the present inventors infected Vero E6 cells with SARS-CoV-2, treated the cells with the complexed, mixed or combined preparation of efavirenz, fluoxetine and the biguanide-based compound, metformin, and then conducted qPCR. The result showed that treatment with the complexed, mixed, or combined preparation of efavirenz and fluoxetine exhibits much more effective antiviral efficacy, compared to treatment with efavirenz, fluoxetine or metformin alone, treatment with the complexed, mixed, or combined preparation of metformin and efavirenz, or treatment with the complexed, mixed, or combined preparation of metformin and fluoxetine. In addition, the result showed that treatment with the complexed, mixed, or combined preparation of efavirenz, fluoxetine and metformin exhibits more effective antiviral efficacy.

In addition, Vero E6 cells were infected with HCoV-OC43, SEOV, VACV and ZIKV, treated with the complexed, mixed or combined preparation of efavirenz and fluoxetine, and then were subjected to qPCR. The result showed that superior antiviral effects can be obtained.

In the present invention, the subject is a mammal in need of treatment for a viral infectious disease. Typically, the subject is a patient infected with a human virus. In one embodiment of the present invention, the subject includes non-human mammals such as non-human primates, animals used in model systems (e.g., mice and rats used for screening, characterization, and evaluation of pharmaceuticals), and other mammals such as rabbits, guinea pigs, hamsters, dogs, cats, monkeys, and anthropoid animals such as chimpanzees and gorillas.

In one embodiment of the present invention, the pharmaceutical composition may be used alone or in combination with surgery, hormone therapy, drug therapy, and biological response regulators to treat patients with viral infections.

The present invention provides the use of a complexed, mixed or combined preparation containing efavirenz and fluoxetine, as a pharmaceutical composition for preventing or treating viral infectious diseases, and the use of a complexed, mixed or combined preparation containing efavirenz, fluoxetine, and containing a biguanide-based compound or a pharmaceutically acceptable salt thereof, as a pharmaceutical composition for preventing or treating viral infectious diseases.

Hereinafter, the present invention will be described in detail with reference to Examples and Experimental Examples.

However, the following Examples and Experimental Examples are provided only for illustration of the present invention and should not be construed as limiting the scope of the present invention.

### <Example 1> Cell culture and virus infection

Vero E6 cells (ATCC No. CRL-1586, ATCC, USA) were incubated at 37°C under 5% CO₂ in Dulbecco's modified eagle medium (DMEM) supplemented with 10% fetal bovine serum (FBS), 1% penicillin/streptomycin and L-glutamine.

When the confluency of the Vero E6 cells in the culture vessel reached about 70 to 80% after subculture in <Example 1>, the required dose was calculated and the Vero E6 cells were infected with the serially diluted specific virus under 5% CO₂ at 37°C for 48 hours.

### <Experimental Example 1> Confirmation of antiviral efficacy of efavirenz, fluoxetine and/or biguanide-based compound against SARS-CoV-2

The Vero E6 cells cultured in <Example 1> were infected with type 2 severe acute respiratory syndrome coronavirus (SARS-CoV-2, NCCP No. 43326, KDCA, Korea). 2 hours later, the Vero E6 cells were treated with a stock (1:1) containing 1.5 µM efavirenz, 1 µM fluoxetine, and/or 2.5 mM metformin as the biguanide compound alone or in combination, or treated with dilutions of 1:10 and 1:100 of the stock alone or in combination. After 48 hours, antiviral efficacy was confirmed according to Experimental Example <1-1> below. As a control group, the Mock group was not infected with the virus, and the SARS-CoV-2 group was treated with PBS instead of efavirenz, fluoxetine, and/or metformin.

### <1-1> Confirmation of SARS-CoV-2 proliferation inhibition effect using quantitative real time polymerase chain reaction (qPCR)

The cells of <Experimental Example 1> were dissolved in RLT buffer containing 1% beta-mercaptoethanol and then 50 µl of RNA was extracted in water treated with diethyl pyrocarbonate (DEPC). The N gene of SARS-CoV-2 was amplified by qPCR using a conventional method to analyze the gene expression level.

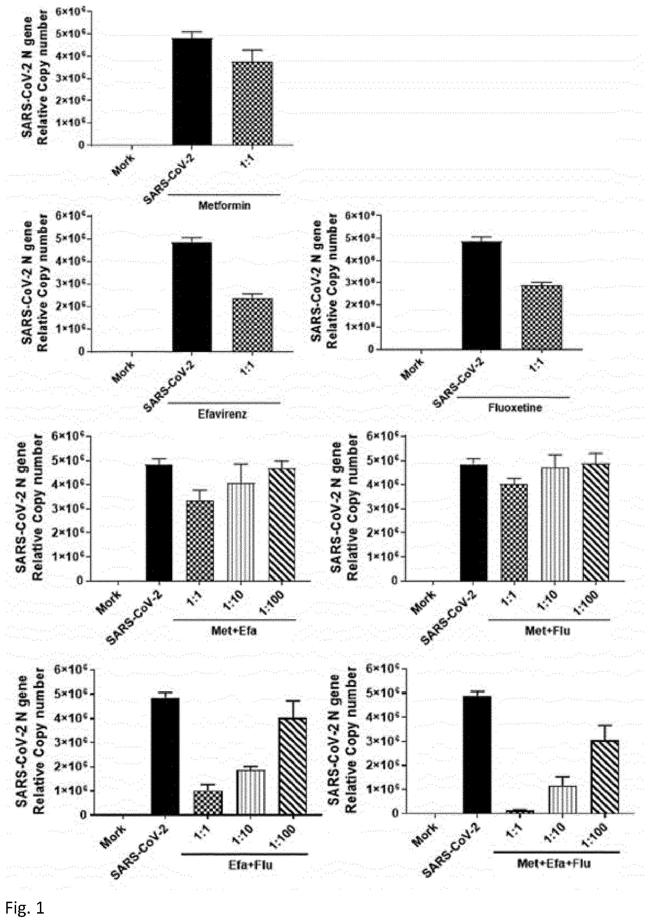

As a result, the group treated with a combination of efavirenz and fluoxetine exhibited a remarkable decrease in the number of genes, compared to the group treated with efavirenz, fluoxetine or metformin alone, the group treated with a combination of efavirenz and metformin, and the group treated with a combination of fluoxetine and metformin. In addition, it was confirmed that the group treated with a combination of efavirenz, fluoxetine, and metformin exhibited superior effect of inhibiting the proliferation of SARS-CoV-2 (FIG. 1).

### <Experimental Example 2> Confirmation of antiviral efficacy of efavirenz and fluoxetine against HCoV-OC43

Antiviral efficacy was confirmed in the same manner as the method of <Experimental Example 1> except that Vero E6 cells cultured in <Example 1> were infected with human coronavirus OC43 (HCoV-OC43), and 2 hours later, the Vero E6 cells were treated with a stock solution containing a combination of 1.5 µM efavirenz and 1 µM fluoxetine. The control group was treated with PBS instead of efavirenz and fluoxetine.

### <2-1> Confirmation of HCoV-OC43 proliferation inhibition effect using qPCR

The gene expression level was analyzed in the same manner as the method of Experimental Example <1-1>, except that the cells of <Experimental Example 2> were used.

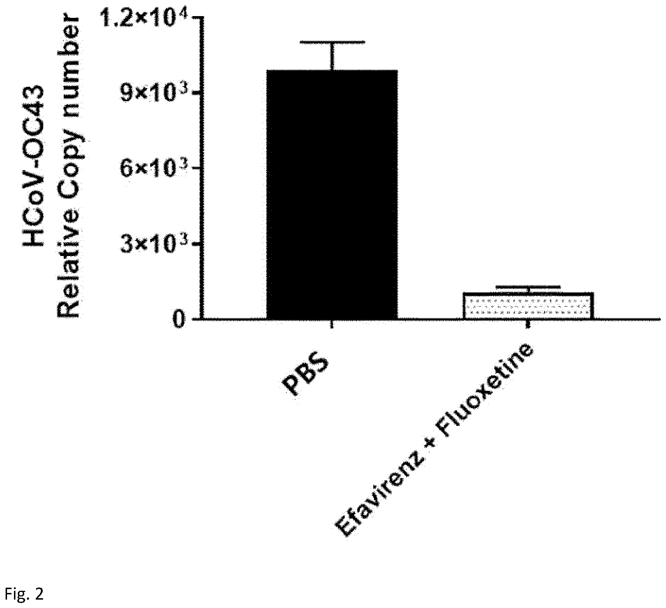

The result showed that the relative gene number of the group treated with the combination of efavirenz and fluoxetine was significantly reduced, compared to the relative gene number of the control group treated with PBS. This indicates that efavirenz and fluoxetine have excellent HCoV-OC43 proliferation inhibitory effects (FIG. 2).

### <Experimental Example 3> Confirmation of antiviral efficacy of efavirenz and fluoxetine against SEOV

Antiviral efficacy was confirmed in the same manner as the method of <Experimental Example 1> except that Vero E6 cells cultured in <Example 1> were infected with *Seoul orthohantavirus* (SEOV), which belongs to the genus of *Orthohantavirus,* and 2 hours later, the Vero E6 cells were treated with a stock solution containing a combination of 1.5 µM efavirenz and 1 µM fluoxetine. The control group was treated with PBS instead of efavirenz and fluoxetine.

### <3-1> Confirmation of SEOV proliferation inhibition effect using qPCR

The gene expression level was analyzed in the same manner as the method of Experimental Example <1-1>, except that the cells of <Experimental Example 3> were used.

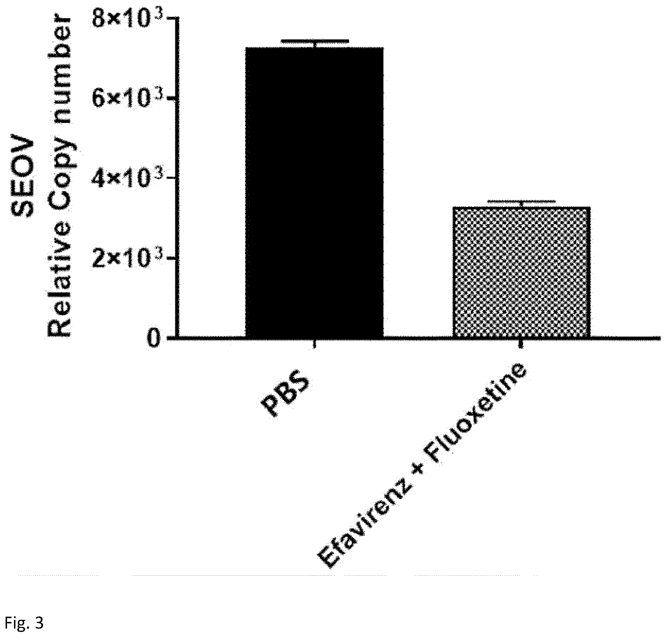

The result showed that the relative gene number of the group treated with the combination of efavirenz and fluoxetine was significantly reduced compared to the relative gene number of the control group treated with PBS. This indicates that efavirenz and fluoxetine have excellent SEOV proliferation inhibitory effects (FIG. 3).

### <Experimental Example 4> Confirmation of antiviral efficacy of efavirenz and fluoxetine against VACV

Antiviral efficacy was confirmed in the same manner as the method of <Experimental Example 1> except that Vero E6 cells cultured in <Example 1> were infected with Vaccinia virus (VACV or VV), which belongs to the genus of *Orthopoxvirus,* and 2 hours later, the Vero E6 cells were treated with a stock solution containing a combination of 1.5 µM efavirenz and 1 µM fluoxetine. The control group was treated with PBS instead of efavirenz and fluoxetine.

### <4-1> Confirmation of VACV proliferation inhibition effect using qPCR

The gene expression level was analyzed in the same manner as the method of Experimental Example <1-1>, except that the cells of <Experimental Example 4> were used.

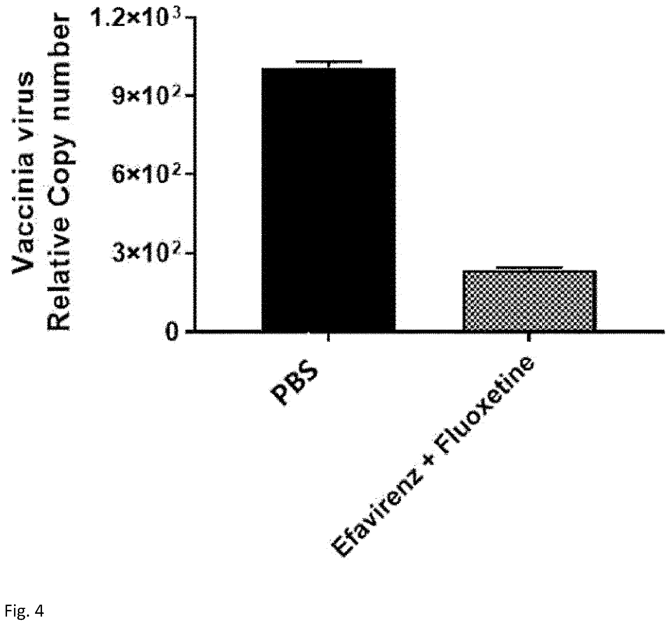

The result showed that the relative gene number of the group treated with the combination of efavirenz and fluoxetine was significantly reduced compared to the relative gene number of the control group treated with PBS. This indicates that efavirenz and fluoxetine have excellent VACV proliferation inhibitory effects (FIG. 4).

### <Experimental Example 5> Confirmation of antiviral efficacy of efavirenz and fluoxetine against ZIKV

Antiviral efficacy was confirmed in the same manner as the method of <Experimental Example 1> except that Vero E6 cells cultured in <Example 1> were infected with *Zika virus* (ZIKV), which belongs to the genus of *Flavivirus,* and 2 hours later, the Vero E6 cells were treated with a stock solution containing a combination of 1.5 µM efavirenz and 1 µM fluoxetine. The control group was treated with PBS instead of efavirenz and fluoxetine.

### <5-1> Confirmation of ZIKV proliferation inhibition effect using qPCR

The gene expression level was analyzed in the same manner as the method of Experimental Example <1-1>, except that the cells of <Experimental Example 5> were used.

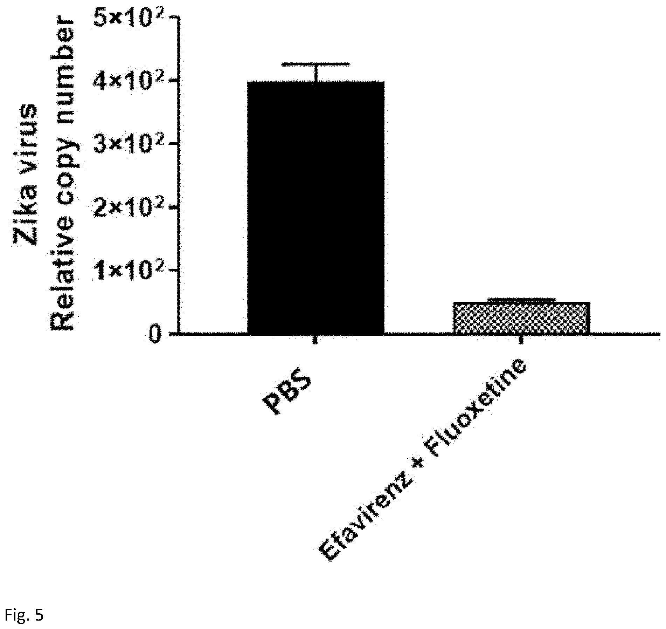

The result showed that the relative gene number of the group treated with the combination of efavirenz and fluoxetine was significantly reduced compared to the relative gene number of the control group treated with PBS. This indicates that efavirenz and fluoxetine have excellent ZIKV proliferation inhibitory effects (FIG. 5).

### [Industrial Applicability]

Treatment with a complexed, mixed, or combined preparation of efavirenz and fluoxetine, or treatment with a complexed, mixed, or combined preparation of metformin, as a biguanide compound, as well as efavirenz and fluoxetine exhibits significantly effective antiviral efficacy against zoonotic viruses, including SARS-CoV-2, compared to treatment with efavirenz, fluoxetine or metformin alone. This indicates that efavirenz, fluoxetine, and the biguanide compound may be useful as an active ingredient of compositions for preventing or treating viral infectious diseases.

## Claims

1. A pharmaceutical composition for preventing or treating viral infectious diseases comprising a first ingredient containing efavirenz and a second ingredient containing fluoxetine as active ingredients, as a complexed, mixed or combined preparation for preventing or treating viral infectious diseases.

2. A pharmaceutical composition for preventing or treating viral infectious diseases comprising a first ingredient containing efavirenz, a second ingredient containing fluoxetine, and a third ingredient containing a biguanide-based compound or a pharmaceutically acceptable salt thereof as active ingredients, as a complexed, mixed or combined preparation for preventing or treating viral infectious diseases.

3. The pharmaceutical composition according to claim 2, wherein the biguanide-based compound is selected from the group consisting of metformin, phenformin, buformin, and biguanide.

4. The pharmaceutical composition according to claim 1, wherein a ratio of the first ingredient containing efavirenz to the second ingredient containing fluoxetine is 0.0000001 to 10 parts by weight : 1 part by weight.

5. The pharmaceutical composition according to claim 2, wherein a ratio of the first ingredient containing efavirenz to the second ingredient containing fluoxetine to the third ingredient containing the biguanide-based compound or the pharmaceutically acceptable salt thereof is 0.0000001 to 10 parts by weights : 1 part by weight : 0.01 to 100000 parts by weights.

6. The pharmaceutical composition according to claim 1 or 2, wherein the virus is selected from the group consisting of *Betacoronavirus* and a subgenus thereof, *Orthohantavirus, Orthopoxvirus, Flavivirus, Lyssavirus* and *Bandavirus.*

7. The pharmaceutical composition according to claim 6, wherein the virus is selected from the group consisting of *Bovine coronavirus, human coronavirus* OC43 (HCoV-OC43), *China Rattus coronavirus* (HKU24), human coronavirus HKU1 (HCoV-HKU1), *murine coronavirus* (M-CoV), *Myodes coronavirus* (2JL14), *severe acute respiratory syndrome coronavirus* 2 (SARS-CoV-2) and variants thereof, *middle east respiratory syndrome coronavirus* (MERS-CoV), *severe acute respiratory syndrome coronavirus* 1 (SARS-CoV-1), severe acute respiratory syndrome-related coronavirus (SARSr-CoV), *Bat SARS-like coronavirus* WIV1 (bat SL-CoV-WIV1), *bat coronavirus* (RaTG13), *hedgehog coronavirus* 1, *Pipistrellus bat coronavirus* (HKU5), *Tylonycteris bat coronavirus* (HKU4), *Eidolon bat coronavirus* (C704), *Rousettus bat coronavirus* (GCCDC1), *Rousettus bat coronavirus* HKU9 (HKU9-1), *Bat Hp-betacoronavirus* Zhejiang2013, *Andes orthohantavirus* (ANDV), *Asama orthohantavirus* (ASAV), *Asikkala orthohantavirus* (ASIV), *Bayou orthohantavirus* (BAYV), *Black Creek Canal orthohantavirus* (BCCV), *Bowe orthohantavirus* (BOWV), *Bruges orthohantavirus, Cano Delgadito orthohantavirus* (CADV), *Cao Bang orthohantavirus, Choclo orthohantavirus* (CHOV), *Dabieshan orthohantavirus, Dobrava-Belgrade orthohantavirus* (DOBV), *El Moro Canyon orthohantavirus, Fugong orthohantavirus, Fusong orthohantavirus, Hantaan orthohantavirus* (HTNV), *Jeju orthohantavirus, Kenkeme orthohantavirus, Isla Vista virus* (ISLAV), *Khabarovsk orthohantavirus* (KHAV), *Laguna Negra orthohantavirus* (LANV), *Muleshoe virus* (MULV), *Luxi orthohantavirus, Maporal orthohantavirus, Montano orthohantavirus* (MTNV), *Necocli orthohantavirus, Oxbow orthohantavirus* (OXBV), *New York virus (*NYV), *Prospect Hill orthohantavirus* (PHV), *Puumala orthohantavirus* (PUUV), *Robina orthohantavirus, Rockport orthohantavirus* (RKPV), *Rio Mamore virus* (RIOMV), *Rio Segundo virus* (RIOSV), *Sangassou orthohantavirus* (SANGV), *Seewis orhtohantavirus, Seoul orthohantavirus* (SEOV), *Sin Nombre orthohantavirus* (SNV), *Tatenale orthohantavirus, Tigray orthohantavirus, Thailand orthohantavirus* (THAIV), *Thottapalayam virus* (TPMV), *Topografov virus* (TOPV), *Tula orthohantavirus* (TULV), *Yakeshi orthohantavirus, Abatino macacapox virus, Akhmeta virus, Alaskapox virus,* Camelpox virus (CMLV), *Cowpox virus, Ectromelia virus, Monkeypox virus, Raccoonpox virus, Skunkpox virus, Taterapox virus, Vaccinia virus, Variola virus, Volepox virus, Apoi virus, Aroa virus, Bagaza virus, Banzi virus, Bouboui virus, Bukalasa bat virus, Cacipacore virus, Carey Island virus, Cowbone Ridge virus, Dakar bat virus, Dengue virus* (DENV), *Edge Hill virus, Entebbe bat virus, Gadgets Gully virus* (GGYV), *Ilheus virus, Israel turkey meningoencephalomyelitis virus, Japanese encephalitis virus* (JE), *Jugra virus, Jutiapa virus, Kadam virus, Kedougou virus* (KEDV), *Kokobera virus, Koutango virus, Kyasanur Forest disease virus* (KFD), *Langat virus* (LGTV), *Louping ill virus, Meaban virus, Modoc virus* (MODV), *Montana myotis leukoencephalitis virus, Murray Valley encephalitis virus* (MVEV), *Ntaya virus, Omsk hemorrhagic fever virus, Phnom Penh bat virus, Powassan virus* (POWV), *Rio Bravo virus, Royal Farm virus, Saboya virus, Saint Louis encephalitis virus, Sal Vieja virus, San Perlita virus, Saumarez Reef virus, Sepik virus* (SEPV), *Tembusu virus, Tick-borne encephalitis virus* (TBEV), *Tyuleniy virus, Uganda 5 virus, Usutu virus* (USUV), *Wesselsbron virus, West Nile virus* (WNV), *Yaounde virus, Yellow fever virus, Yokose virus* (YOKV), *Zika virus, Rabies lyssavirus,* and severe fever with thrombocytopenia syndrome virus (SFTSV).

8. The pharmaceutical composition according to claim 1 or 2, wherein the pharmaceutical composition is formulated as a formulation selected from the group consisting of a tablet, a capsule, an injection, a troche, a powder, a granule, a solution, a suspension, an oral liquid, an emulsion, a syrup, a suppository, a vaginal tablet, and a pill.

9. A complexed, mixed or combined preparation kit for preventing or treating viral infectious diseases comprising a preparation containing efavirenz and a preparation containing fluoxetine.

10. A complexed, mixed or combined preparation kit for preventing or treating viral infectious diseases comprising a preparation containing efavirenz, a preparation containing fluoxetine, and a preparation containing a biguanide-based compound or a pharmaceutically acceptable salt thereof.

11. A complexed, mixed or combined preparation kit for preventing or treating viral infectious diseases according to claim 9 or 10, wherein the complexed, mixed or combined preparation is formulated as a formulation selected from the group consisting of a tablet, a capsule, an injection, a troche, a powder, a granule, a solution, a suspension, an oral liquid, an emulsion, a syrup, a suppository, a vaginal tablet, and a pill.

12. A method of preventing or treating viral infectious diseases comprising administering a complex, mixture or combination of a first ingredient containing efavirenz and a second ingredient containing fluoxetine to a subject.

13. A method of preventing or treating viral infectious diseases comprising administering a complex, mixture or combination of a first ingredient containing efavirenz, a second ingredient containing fluoxetine, and a third ingredient containing a biguanide-based compound or a pharmaceutically acceptable salt thereof to a subject.

14. Use of a complexed, mixed or combined preparation containing a first ingredient containing efavirenz and a second ingredient containing fluoxetine as active ingredients, as a pharmaceutical composition for preventing or treating viral infectious diseases.

15. Use of a complexed, mixed or combined preparation comprising a first ingredient containing efavirenz, a second ingredient containing fluoxetine, and a third ingredient containing a biguanide-based compound or a pharmaceutically acceptable salt thereof as active ingredients, as a pharmaceutical composition for preventing or treating viral infectious diseases.
